# EUROPEAN PATENT SPECIFICATION

(11) **EP 3 110 850 B1**
(45) Date of publication and mention of the grant of the patent: **21.11.2018**
(21) Application number: 15755150.8
(22) Date of filing: 27.02.2015
(51) Int. Cl.: C08B 1/00, C07C 211/63, C01B 35/10, B01J 20/24, C08J 9/28

(54) **DEEP EUTECTIC SOLVENTS AND THEIR USE**
TIEFE EUTEKTISCHE LÖSUNGSMITTEL UND DEREN VERWENDUNG
SOLVANTS EUTECTIQUES PROFONDS ET LEUR UTILISATION

(30) Priority: 28.02.2014 FI 20145195
(43) Date of publication of application: 04.01.2017
(73) Proprietor: Teknologian tutkimuskeskus VTT Oy, 02044 VTT (FI)
(72) Inventor: HILTUNEN, Jaakko, FI-02044 Espoo (FI); VUOTI, Sauli, FI-02044 Espoo (FI); KUUTTI, Lauri, FI-02044 Espoo (FI)
(74) Representative: Boco IP Oy Ab
(86) International application number: PCT/FI2015/050122
(87) International publication number: WO 2015/128550

(56) References cited:
- WO-A1-2013/153203
- WO-A1-2014/131906
- WO-A2-2009/085574
- WO-A2-2012/145522
- JP-A- 2000 063 463
- US-A1- 2009 247 432
- GAO, Y. ET AL.: 'Electrodeposition of SnBi coatings based on deep eutectic solvent' SURFACE ENGINEERING vol. 30, no. 1, 2014, ISSN 0267-0844 pages 59 - 63, XP055219888

## Description

### FIELD OF THE INVENTION

The present invention relates to deep eutectic solvents, to their manufacture and use in the dissolution of polysaccharides, in the manufacture of cellulose beads, in the manufacture of cellulose absorbent, and to a method for dissolving polysaccharides and/or natural polyphenols, to a method for the manufacture of cellulose beads and to a method for the manufacture of cellulose absorbent.

### BACKGROUND OF THE INVENTION

An ionic liquid is a salt in the liquid state. Typically ionic liquids refer to salts having melting point below 100°C. Ordinary liquids, such as water and gasoline are predominantly composed of neutral molecules, but ionic liquids are made of ions and short lived ion-pairs.

Deep eutectic solvents (DES) are a sub-class of ionic liquids with special properties. DES is composed of a mixture which forms a eutectic with a melting point much lower than any of the individual components. The first generation eutectic solvents were based on mixtures of quaternary ammonium salts with hydrogen donors, such as amines and carboxylic acids. The deep eutectic phenomenon was first described in 2003 for a mixture of choline chloride and urea in a 1:2 mole ratio, respectively. Choline chloride has a melting point of 302°C and that of urea is 133°C. The eutectic mixture melts at 12°C.

A eutectic mixture formed of choline chloride and urea has been studied in cellulose modifications and dissolution, however only modest concentrations of cellulose of less than 4 wt% were dissolved in said mixture. Also combinations of choline chloride with malonic acid or glycerol have been tested, but so far no mixtures have been reported, which are able to dissolve more than 5 wt% of cellulose.

Typically many polysaccharides are difficult to dissolve. An example of such polysaccharides is cellulose, which is used for example as starting material in the manufacture of regenerated cellulose. Particularly homogeneous processing of cellulose is challenging due to low or negligible solubility of cellulose in water or most organic solvents. Various methods have been developed for dissolving cellulose and the main industrial processes for the manufacture of regenerated cellulose via cellulose dissolution are the traditional derivatization viscose process based cellulose xanthate derivative, and the non-derivatization (Lyocell) process based on interaction with NMMO (N-methyl-morpholine-N-oxide). Both of these methods are associated with environmental, technical and/or economical disadvantages.

Also ionic solvents have been proposed for dissolving lignocellulosic materials, but they are often harmful or toxic and very expensive for industrial use on a larger scale. Thus improved methods and solvent systems for dissolving cellulose are needed.

Cellulose beads are spherical particles with diameters in the micro- to millimeter scale. Cellulose beads are used in many advanced applications ranging from chromatography over solid supported synthesis and protein immobilization to controlled drug release. Over the last decades, various procedures for the preparation of cellulose beads have been reported, including the use of different solvents, shaping techniques, and technical devices for large batch production. Functional materials for specific applications have been prepared by introducing numerous chemical functionalities or blending cellulose with organic and inorganic compounds. In addition, commercial cellulose bead products with defined properties are available. However, the methods for producing the beads have involved the use of complicated solubilization processes for cellulose, such as freezing the material and solubilizing in NaOH/urea mixtures or ionic liquids. Additionally, these methods have demanded laborious pre-treatment methods such as acid hydrolysis, homogenization etc. The price of cellulose beads manufactured by these methods is typically very high, thus limiting their use.

Because of the growth of offshore oil production and transportation, the probability of oil spill accidents has dramatically increased over the past several decades. Conventional methods currently employed for oil spill cleanup typically involve (i) dispersing the oil phase in water with the help of dispersing agents to facilitate natural degradation, (ii) burning the floating oil in situ, and (iii) extracting the oil phase from the water surface using a sorbent. Among these alternatives, the removal of oil by sorbent materials is considered to be the most efficient, economical, and ecological as the pollutant can be properly discarded and no secondary pollution is created. Several criteria should be met for a material to be used as an oil absorbent, including high oil absorption capacity and oil/water selectivity, fast oil sorption rate, high floatability (hence a low density), low cost, environmentally friendliness, and recyclability.

A wide range of sorbents have been proposed so far, from organic synthetic products elaborated from polypropylene or polystyrene to inorganic mineral materials, including organophilic clays, silica aerogels, and exfoliated graphite. However, the oil absorption capacity of organic absorbents is generally limited, and the production costs associated with inorganic sorbents, such as silica aerogels and exfoliated graphite remain too high to envisage their widespread application as depolluting agents. Moreover, none of these materials is biodegradable. There is a clear need for cheap and biodegradable oil adsorbents that are water-insoluble.

Despite the ongoing research and development there is a need for more effective solvent systems and particularly eutectic solvents, having improved dissolving properties, low toxicity and low cost. There is also a need for improved processes for improved processes for the dissolution of polysaccharides.

### SUMMARY OF THE INVENTION

An object of the invention is to provide new deep eutectic solvents.

Another object of the invention is to provide a method for the manufacture of the deep eutectic solvents.

Another object of the invention is to provide uses of said new deep eutectic solvents, particularly in the field of dissolution of polysaccharides and/or natural polyphenols.

A still another object of the invention is to provide a method where polysaccharides can be dissolved effectively and economically.

Thus the present invention relates to new deep eutectic solvents comprising a (2-R-ethyl)-trimethylammonium salt or a mixture of said salts, and a Lewis acid selected from boric acid, meta-boric acid, boronic acid, borinic acid, alkyl borates and hydrated borate salts.

The present invention also relates to the method for the manufacture of the deep eutectic solvents.

The present invention also relates to the use of the new deep eutectic solvents in the dissolution of polysaccharides and/or natural polyphenols.

The present invention also relates to a method for dissolving polysaccharides and/or natural polyphenols.

The present invention also relates to a method for the manufacture of cellulose beads, which method comprises the steps where 0.1 - 50 wt% of cellulosic material is mixed with the deep eutectic solvent according to any one of claims 1-7, to obtain a cellulosic suspension, followed by adding 1-10 wt% of water and mixing to obtain an aqueous mixture, followed by forcing the aqueous mixture through a nozzle drop-wise to an aqueous nitric acid solution whereby cellulose beads are formed, and washing the cellulose beads with water.

The present invention also relates to a method for the manufacture of cellulose absorbent, which method comprises the steps where 1-50 wt% of cellulosic material is mixed with the deep eutectic solvent according to any one of claims 1-7, to obtain a homogeneous cellulosic suspension, followed by adding water to the suspension, whereby a mixture comprising precipitation is formed, followed by filtrating the mixture and washing with water and freeze-drying the obtained solid material.

Characteristic features of the invention are presented in the appended claims.

### DEFINITIONS

The term "(2-R-ethyl)-trimethylammonium salt" refers here to compound having chemical formula I below, including inorganic and organic salts of said compound. R is selected from OH, halogens, ester groups, ether groups and carbamoyl group.

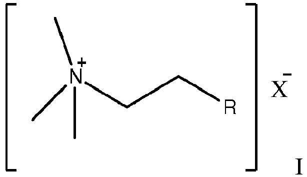

Choline chloride, known also as 2-hydroxy-*N*,*N*,*N*-trimethylethanaminium chloride or 2-hydroxyethyl-trimethylammonium chloride, is an example of said compounds and it has the following chemical formula II:

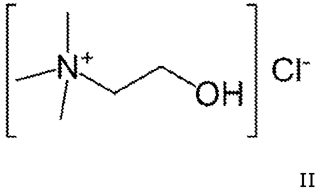

The term "boric acid" refers here to H₃BO₃, an inorganic Lewis acid having melting point of 170.9°C. It is also called as hydrogen borate, boracic acid, ortho-boric acid and *acidum boricum*.

The term "betaine" refers here to N,N,N-trimethyl glycine and salts thereof, having the following chemical formula III:

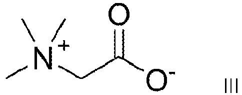

The term cellulose or polysaccharide or polyphenol "dissolution" refers here to solubilization or dissolving of cellulose or polysaccharide or polyphenol, where at least part of said cellulose or polysaccharide or polyphenol is solubilized or dissolved and a solution and/or suspension is formed.

The term "cellulose solution" refers here to cellulose dope or cellulose suspension or homogeneous viscoelastic suspension or specific lyotropic liquid crystal solution or isotropic solution.

### DETAILED DESCRIPTION OF THE INVENTION

It was surprisingly found that a (2-R-ethyl)-trimethylammonium salt, or a mixture of said salts, forms a DES with a Lewis acid selected from boric acid, meta-boric acid, boronic acid, borinic acid, alkyl borates and hydrated borate salts and any combinations thereof, even without any added water.

### DES

The DES comprises (2-R-ethyl)-trimethylammonium salt, or a mixture of said salts, and a Lewis acid selected from boric acid, meta-boric acid, boronic acid, borinic acid, alkyl borates and hydrated borate salts, and any combinations thereof.

In the (2-R-ethyl)-trimethylammonium salt the group R is selected from OH, halogens, ester groups, ether groups and carbamoyl group. The halogens are selected from F, CI, I and Br; the ester groups are suitably selected form formyl, acetyl, isopropyl and butyryl groups. Preferably the R is OH, CI, acetyl or formyl group.

The counter anion (X) in the salt can be an inorganic or organic counter anion. The inorganic salt is suitably a halogenide, sulphate or phosphate, preferably chloride salt. The organic salt is suitably acetate, lactate, butyrate or formiate.

Preferably the (2-R-ethyl)-trimethylammonium salt is selected from choline chloride, acetylcholine chloride, and chlorocholine chloride. Choline chloride is a widely available material and it is used for example as low cost animal feed.

The DES comprises a mol ratio from 5:1 to 1:1, preferably from 3:1 to 1:1 of the (2-R-ethyl)-trimethylammonium salt, or a mixture of said salts, and a Lewis acid, respectively.

Optionally the DES may comprise water and/or betaine compound.

The DES may comprise 0-50 wt%, preferably 0.01-50 wt%, more preferably 2-15 wt% of water, calculated from the total weight of the DES. The amount of water may be decreased for example by distillation, evaporation etc. or it may be increased during the manufacture of DES.

The DES may comprise 0-50 wt%, preferably 0.01-50 wt%, more preferably 0.1-10 wt% and particularly preferably 0.1-3 wt% of a betaine compound, calculated from the total weight of the DES. Said betaine compound is selected from betaine and betaine aldehyde. The betaine compound may also be used as salt, such as betaine hydrochloride, and as betaine monohydrate and as anhydrous betaine..

The pH of the DES is suitably at least 7.

### Method for the manufacture of DES

The method for the manufacture of said DES comprises the steps, where a (2-R-ethyl)-trimethylammonium salt or a mixture of said salts is mixed with a Lewis acid selected from boric acid, , meta-boric acid, boronic acid, borinic acid, alkyl borates and hydrated borate salts, and any combinations thereof.

In the (2-R-ethyl)-trimethylammonium salt the group R is selected from OH, halogens, ester groups, ether groups and carbamoyl group. The halogens are selected from F, CI, I and Br; the ester groups are suitably selected form formyl, acetyl, isopropyl and butyryl groups. Preferably the R is OH, CI, acetyl or formyl group.

The counter anion (X) in the salt can be an inorganic or organic counter anion. The inorganic salt is suitably a halogenide, sulphate or phosphate, preferably chloride salt. The organic salt is suitably acetate, lactate, butyrate or formiate.

Preferably the (2-R-ethyl)-trimethylammonium salt is selected from choline chloride, acetylcholine chloride, and chlorocholine chloride.

In the method the amounts of the components are selected to provide a mol ratio from 5:1 to 1:1, preferably from 3:1 to 1:1 of the (2-R-ethyl)-trimethylammonium salt and the Lewis acid, respectively.

Optionally water may be added at any stage of the method, before or after mixing the (2-R-ethyl)-trimethylammonium salt with the Lewis acid. 0-50 wt%, preferably 0.01-50 wt%, more preferably 2-15 wt% of water, calculated from the total weight of the DES is added. The water may be tap water, ion exchanged water or purified water. Water may optionally be added in step-wise manner, preferably 50 wt% of water is added in the first step followed by mixing and adding the remaining 50 wt% in at least one step followed by mixing. The last added amount of water may be 2 wt%.

Optionally a betaine compound is added to the mixture of the (2-R-ethyl)-trimethylammonium salt with a Lewis acid. Said betaine compound is selected from betaine and betaine aldehyde. 0-50 wt%, preferably 0.01-50 wt%, more preferably 0.1-10 wt% and particularly preferably 0.1-3 wt% of the betaine compound, calculated from the total weight of the DES is added.

Betaine may be used as betaine hydrochloride or betaine monohydrate or as betaine anhydrate.

In the method the mixing may be carried out at a temperature of 20-100°C, preferably 40-95°C, particularly preferably 50-90°C. If no water is used, it may be necessary to use temperatures of at least 50°C, particularly when the moe ratio of the (2-R-ethyl)-trimethylammonium salt and the Lewis acid is from 3:2 to 1:1, respectively. It may also be necessary to keep the obtained DES at the temperature of at least 40°C for maintaining it in solution. The mixing may be carried out under a pressure from 100 mbar to 10 bar, suitably normal atmospheric pressure is used.

The pH of the DES may optionally be adjusted with an organic or inorganic base to pH at least 7.

### Method for dissolving of polysaccharides and/or natural polyphenols

The present inventors found that the DES of the invention can be used for dissolving polysaccharides and/or natural polyphenols. Said DES, based on (2-R-ethyl)-trimethylammonium salt or a mixture of said salts, and a Lewis acid selected from boric acid, meta-boric acid, boronic acid, borinic acid, alkyl borates and hydrated borate salts, and any combinations thereof, is cost effective and it has good dissolving efficiency.

Dissolving of polysaccharides and/or natural polyphenols refers here to dissolving in general, but it includes also opening of the fiber structure, swelling, fibrillation and selective removal of hemicellulose. Thus some fibrilles may also be found in the dissolved polysaccharide product.

Polysaccharides, such as cellulose can be dissolved using the DES according to the invention, whereby regenerated cellulose can be produced without the need to use the NNMO or viscose processes. Man-made fiber from wood-based cellulose is considered as a sustainable alternative for cotton. The use of wood-based raw materials reduces sweet water consumption, particularly for irrigation, as well as occupation of agricultural land and the use of plant protection chemicals. This method for dissolving cellulose is environmentally friendly, economic and efficient way of providing dissolved cellulose. The dissolved cellulose may be further processed to various products, for example to cellulose fibers with methods known from viscose processing. Cellulose may be restored from the solution by adding water to the solution whereby cellulose is precipitated.

The obtained solutions may be isotropic or anisotropic, depending on the temperature and the content of the polysaccharide, such as cellulose contained in the solution.

The method for dissolving polysaccharides comprises the steps, where 0.1 - 50 wt% of a polysaccharide is mixed with 99.9 - 50 wt% of a DES according to the invention comprising (2-R-ethyl)-trimethylammonium salt or a mixture of said salts, and a Lewis acid selected from boric acid, meta-boric acid, boronic acid, borinic acid, alkyl borates and hydrated borate salts, and any combinations thereof, at a temperature of 20-150°C to obtain a polysaccharide solution. Said dissolving may be carried out under the pressure from 100 mbar to 10 bar. Typically a clear solution is obtained.

Preferably 0.5-30 wt% of the polysaccharide is used in the method.

The polysaccharide may comprise water. After or during the solubilization of the polysaccharide in the DES at least part of water may be removed using methods known in the art, such as evaporation.

The DES may comprise 0-50 wt%, preferably 0.01-50 wt%, more preferably 2-15 wt% of water, calculated from the total weight of the DES. Water may prevent potential side reactions. The amount of water can be varied during the dissolution process.

The DES may comprise 0-50 wt%, preferably 0.01-50 wt%, more preferably 0.1-10 wt% and particularly preferably 0.1-3 wt% of a betaine compound selected from betaine and betaine aldehyde, calculated from the total weight of the DES.

In the case chlorocholine chloride is used as an additional (2-R-ethyl)-trimethylammonium salt, it may be used in molar excess of 300-400% with respect to the polysaccharide.

Preferably the mixing temperature is 50-90°C.

Preferably the mixing is carried out for 15 min - 24 hours.

The polysaccharide is suitably ground or milled prior to the dissolution step to obtain smaller particles, where by the dissolution time is shorter.

The polysaccharide is suitably selected from cellulose, cellulose derivatives, starch, starch derivatives, xylanes, hemicelluloses, chitosans, pectins, maltodextrines, dextranes and lignocellulosic materials. Preferably polysaccharides are dissolved.

Natural polyphenols are selected from lignins and tannins.

Optionally the betaine compound may be added to the mixture of the DES and the polysaccharide, particularly in cases where the DES does not contain a betaine compound. The amount of the added betaine compound is 0.1-20 wt%, preferably 0.1 - 10 wt%, calculated from the mixture.

Alternatively betaine may be added as dry substance or as aqueous solution before or after the addition of the polysaccharide.

The betaine compound may be used for enhancing the dissolution of the polysaccharide.

The dissolved polysaccharide and/or natural polyphenols may be separated by precipitation with lower alcohol (C1-C5 alcohol), at temperatures below 25°C, suitably less than 10°C, whereby a precipitated product is obtained. Said product may be dried to obtain a powdery product.

Optionally the pH of the mixture comprising the DES and polysaccharide and/or natural polyphenols, and optionally water and/or betaine, may be adjusted to about 7-9 with an organic or inorganic base, such as alkali metal hydroxides or borates, alkaline earth metal hydroxides or borates, ammonium hydroxide, ammonium borate, ammonia gas, DBU (diazabicyclo undecene) pyridine, triethylamine or imidazole. When the betaine compound is used as a salt (hydrochloride), it is advantageous to liberate betaine with a base.
Optionally the pH of the mixture comprising the DES and optionally water and/or betaine may be adjusted to about 7-9 with an organic or inorganic base prior to the addition of the polysaccharide and/or natural polyphenols.

Optionally water or a lower alcohol (C1-C5 alcohol) or mixtures thereof may be added for adjusting the viscosity of the polysaccharide and/or natural polyphenols solution.

The DES according to the invention is particularly suitable for dissolving polysaccharides whereby the dissolved polysaccharides may be further used for the manufacture of regenerated cellulose products, fibers, films, flocculants, cosmetics, sizing compounds, fixatives, cleaning aids, detergents etc. Examples of such applications are film coatings, capsules and devices in drug delivery. Also special fibers comprising boron and suitable for the manufacture of fire-resistant structures and textiles may be obtained with the invention.

The present invention provides several advantages. The new DES is an effective, low cost and environmentally acceptable solvent, which is particularly suitable for dissolving polysaccharides.

The manufacture of said DES is simple, no residues are left and no specific equipment is needed. The boron component is stable, inert and easily recyclable.

The dissolution process for dissolving polysaccharides and/or natural polyphenols may be carried out in a simple manner and it takes place rapidly. The components are recyclable, no harmful emissions are released. The method provides a new concept for providing dissolved cellulose, which may be utilized for example instead of the conventional viscose processes.

The use of the DES of the invention incorporates nitrogen into the polysaccharide, and when additionally a betaine compound is used the nitrogen content in the dissolved polysaccharide may be even 3.4 wt%.

The solutions comprising the dissolved polysaccharides may also be obtained as highly viscous materials, which are particularly suitable for extrusion processes, film manufacture spinning of fibers and the like, where highly viscous materials are processed.

### Method for the manufacture of cellulose beads

The DES of the invention is also particularly useful in the manufacture of cellulose beads.

The method of the invention for the manufacture of cellulose beads comprises the steps, where 0.1 - 50 wt% of cellulosic material is mixed with the deep eutectic solvent of the invention, to obtain a cellulosic suspension, followed by adding 1-10 wt% of water and mixing to obtain an aqueous mixture, followed by forcing the aqueous mixture through a nozzle drop-wise to an aqueous nitric acid solution whereby cellulose beads are formed, and washing the cellulose beads with water.

The method for the manufacture of cellulose beads comprises the steps, where 0.1 - 50 wt% of cellulosic material is mixed with the DES of the invention, at a temperature of 20-150°C. A cellulosic suspension is obtained, which is followed by adding 1-10 wt% of water and mixing at a temperature of 20-150°C to obtain an aqueous mixture. The mixture is forced through a nozzle having an inner diameter in the range of 0.5-2 mm, drop-wise to an aqueous nitric acid solution whereby cellulose beads are formed, and washing the cellulose beads with water. Nitric acid solution is suitably 0.1-5 M solution. Suitably the cellulosic material is solubilized in the DES by warming the mixture at elevated temperature for 5-30 hours. After the water is added the mixture is suitably stirred for 10 min to 5 hours.

According to a preferable embodiment the mixture is forced using increased pressure of 0.1-10 bar.

According to a preferable embodiment the method is carried out at the temperature of 10 - 40°C.

The cellulose beads are suitably left in nitric acid for 30 min to 5 h to ensure complete coagulation, followed by washing. The washing is suitably carried out using tap water and then with distilled water until the pH of the mixture remains constant. After the washing procedure the cellulose beads remain inert to mixing and are not dissociated upon mechanical stirring.

### Cellulosic material

The cellulosic material may comprise a wide variety of cellulose based materials. Pulp derived from wood pulp and pulp from other natural sources may be used, such as kenaf, straw, acaba, coir, flax, jute, kapok, raffia, bamboo, hemp, modal, pine, ramie, sisal etc. may be used. Softwood pulps and hardwood pulps and mixtures thereof are suitable for the present process. Mechanically treated pulp fibers, chemically treated pulp fibers and chemimechanically treated pulp fibers, such as chemithermomechanical pulp fibers (CTMP), bleached chemithermomechanical pulp fibers (BCTMP), thermomechanical pulp fibers (TMP), refiner groundwood pulp fibers, groundwood pulp fibers, enzymatically treated pulp, cellulose, cellulose derivatives, enzymatically treated cellulose, and combinations thereof may be used.

Preferably wood-based lignocellulose materials are used.

The method for the manufacture of cellulose beads according to the invention does not require any acidic pre-treatments, because boric acid is already present in the deep eutectic solvent mixture. The price of the DES is low, and the mixture is environmentally friendly. The method is also economic and simple.

The obtained cellulose beads find use in chromatography, solid supported synthesis and protein immobilization, as well as in encapsulation applications in the field of agricultural products and paint applications.

### Method for the manufacture of cellulose absorbent

The DES of the invention is also particularly useful in the manufacture of cellulose absorbent.

The method according to the invention for the manufacture of cellulose absorbent comprises the steps where 1-50 wt% of cellulosic material is mixed with the deep eutectic solvent according to the invention, to obtain a homogeneous cellulosic suspension, followed by adding water to the suspension, whereby a mixture comprising precipitation is formed, followed by filtrating the mixture and washing with water and freeze-drying the obtained solid material.

The method for the manufacture of cellulose absorbent comprises the steps where 1-50 wt% of cellulosic material is mixed with the deep eutectic according to the invention, at a temperature of 50-120°C to obtain a homogeneous cellulosic suspension. This is followed by adding water to the suspension to disrupt the formulation and to hydrate the cellulose polymers and DES components, whereby a mixture comprising precipitation is formed, followed by filtrating the mixture and washing with water and freeze-drying the obtained solid material.

The cellulosic material may comprise a wide variety of cellulose based materials. Pulp derived from wood pulp and pulp from other natural sources is suitably used, such as kenaf, straw, acaba, coir, flax, jute, kapok, raffia, bamboo, hemp, modal, pine, ramie, sisal etc. may be used. Softwood pulps and hardwood pulps and mixtures thereof are suitable for the present process. Mechanically treated pulp fibers, chemically treated pulp fibers and chemimechanically treated pulp fibers, such as chemithermomechanical pulp fibers (CTMP), bleached chemithermomechanical pulp fibers (BCTMP), thermomechanical pulp fibers (TMP), refiner groundwood pulp fibers, groundwood pulp fibers, enzymatically treated pulp, cellulose, cellulose derivatives, enzymatically treated cellulose, and combinations thereof may be used.

Preferably wood-based lignocellulose materials are used.

In the method excess of water is added to the homogeneous cellulosic suspension to disrupt the formulation and to hydrate the cellulose polymers and DES components, whereby a precipitation is formed. The aqueous mixture is then filtrated and cellulose is subsequently carefully washed to remove residual DES components. Finally, the cellulose material is freeze-dried to obtain cellulose absorbent material.

The cellulose material is preferably washed with water prior to drying.

The drying is preferably carried out using freeze-drying, whereby the dry product comprises a porous and spongy structure. The spongy product is insoluble in water.

Optionally, methyltrimethoxysilane in a molar ratio of 0-10 with regard to anhydroglucose units may be added to the cellulosic suspension prior to the water addition.

A cellulose absorbent with surprisingly good oil adsorbing qualities is obtained.

Dodecane and silica oil are generally used as suitable model compounds for oil adsorbency. Samples of the cellulose absorbent product were tested and the oil absorption capacity was calculated from the mass of the oil adsorbed into the sample. The highest value for absorption capacity was 43.2g (dodecane) and 39.4g (silica oil), with silyl treated sample 50.8g (dodecane).

Oil can be removed from the cellulose absorbent by squeezing or pressing. The cellulose absorbent retains its form, and this can be enhanced with additional drying.

The cellulose absorbent material can be used for absorbing water-immiscible or water insoluble liquids, particularly oils and oily materials. It is reusable, cost-effective and environmentally sustainable material.

The cellulose absorbent material can be also used to collect oil from water and from the surface of water. Water is typically initially slightly adsorbed into the material, but when the material is squeezed lightly, water drips away but oil is mostly still attached in to the sample.

The method for the manufacture of the cellulose absorbent material is cost efficient and simple, there is no need for the use of toxic chemicals or multistep processes. The cellulose absorbent material is highly porous, cellulose-based and biodegradable, and causes no difficulties if used in natural waters.

The invention will now be illustrated with the following examples.

### Examples

### Example 1

### Manufacture of DES comprising choline chloride:boric acid (ratio 3:1 by mol)

14.77 g (0.24 mol) of boric acid and 100.0 g of choline chloride (0.72 mol) were mixed thoroughly with a mechanical laboratory mixer. 5 ml of water (4 wt%) was added to the mixture and the temperature was increased to 60°C. A clear DES solution was formed in 1-2 hours.

### Example 2

### Dissolution of birch pulp cellulose

5.0 g (4 wt%) of freeze-dried birch pulp cellulose (0.03 mol of OH groups) was added to the DES solution obtained in example 1 and temperature was increased to 80°C. A viscous cellulose dope was formed during 3-4 hours.

### Example 3

### Lowering the viscosity of birch pulp cellulose solution

To the cellulose solution obtained in example 2, 1.0 g of sodium hydroxide (25 mmol) dissolved in 1.0 ml of water and 2.0 g of betaine hydrochloride (13 mmol) were added. Viscosity of the solution decreased after one hour of the addition. The product was precipitated using 500ml of cold ethanol, filtered and dialyzed against water for 48 hours with a membrane (cutoff 3000) and freeze-dried, the final cellulose product contained 2.4 wt% of nitrogen as analysed by using Kjehldahl titration.

### Example 4

### Manufacture of DES comprising choline chloride:boric acid (ratio 3:2 by mol)

29.54 g (0.48 mol) of boric acid and 100.0 g of choline chloride (0.72 mol) were mixed thoroughly with a mechanical laboratory mixer. The temperature was increased to 80°C. A clear DES solution was formed during one hour.

### Example 5

### Dissolution of birch pulp cellulose

5.0 g (4 wt%) of freeze-dried birch pulp cellulose (0.03 mol of OH groups) was added to the DES solution obtained in example 4 at 80°C. A viscous solution was formed during 3-4 hours.

### Example 6

### Lowering the viscosity of birch pulp cellulose solution

10.0 g (7 wt%) of cellulose was added to the DES obtained in example 4 and temperature was increased to 80°C. A viscous gel was formed during 3-4 hours. 0.35 g of sodium hydroxide (8.75 mmol) dissolved in 0.5 ml of water and 1.0 g of betaine (8.5 mmol) were added to the mixture. After one hour of mixing at ambient temperature, the viscosity decreased and mixing enhanced remarkably when examined visually. The product was precipitated using 500ml of cold ethanol, filtered and dialyzed against water for 48 hours with a membrane (cutoff 3000) and freeze-dried, the final cellulose product contained 1.57 wt% of nitrogen as analysed by using Kjehldahl titration.

### Example 7

### Dissolution of potato starch

DES was manufactured as instructed in example 1. 14.77 g (0.24 mol) of boric acid and 100.0 g of choline chloride (0.72 mol) were mixed thoroughly with a mechanical laboratory mixer. 5 ml of water was added to the mixture and the temperature increased to 60°C. A clear DES solution was formed in 1-2 hours.

5.0 g (4 wt%) of potato starch (0.03 mol of OH groups) was added to the obtained DES solution and temperature was increased to 80°C. A viscous, clear potato starch solution was formed during 3-4 hours.

### Example 8

### Lowering the viscosity of potato starch solution

To the potato starch solution obtained in example 7, 1.0 g of sodium hydroxide (25.0 mmol) dissolved in 1.0 ml of water and 5.0 g of betaine (42.5 mmol) were added to the mixture. After one hour of mixing at ambient temperature, the viscosity decreased and mixing enhanced remarkably when examined visually. The product was precipitated using 500ml of cold ethanol, filtered and dialyzed against water for 48 hours with a membrane (cutoff 3000) and freeze-dried, the final potato starch product contained 1.3 wt% of nitrogen as analyzed by using Kjehldahl titration.

### Example 9

### Manufacture of DES comprising choline chloride: boric acid (ratio 5:3 by mol)

53.1 g (0.86 mol) of boric acid and 200.0 g of choline chloride (1.43 mol) were put into teflon vessel and mixed thoroughly with a mechanical laboratory mixer at 80°C. A clear DES solution was formed in 1 hour.

### Example 10

### Dissolution of dissolving cellulose pulp in DES of example 9

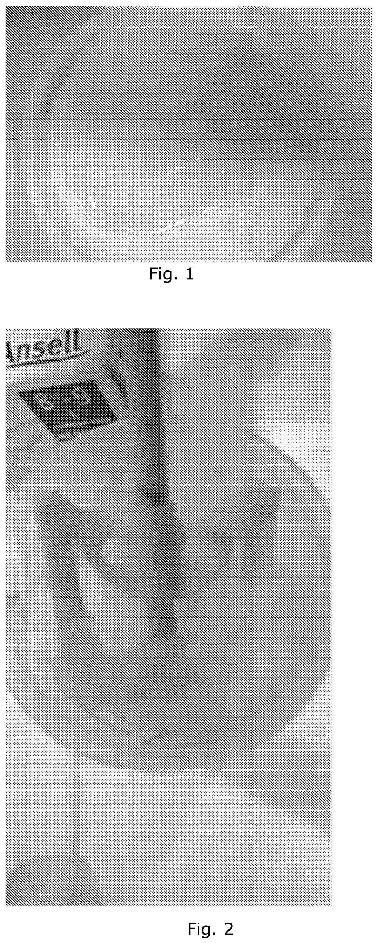

3.8 g of anhydrous betaine (0.028 mol) was dissolved in 4.2 g (0.23 mol) of distilled water. 16.0 g of dried dissolving pulp (dry-matter content∼94 %) was immersed in 16.0 g of distilled water to activate the dry pulp. The betaine solution and activated pulp were added into the DES solution described in example 9. The open vessel was kept in a water-bath at 80°C and the slurry was vigorously stirred. Water was evaporated from the slurry until the water content was approximately 5 wt-% of the total DES solution weight (net weight of water, choline chloride and boric acid). This took approximately 2 hours. The cellulose concentration was approximately 5.3 wt-% of the total mass weight. After this the vessel was sealed and the stirring was continued during 2 hour. A turbid viscous dope containing some fibre particles was formed. In Figure 1 a photo of the obtained dope is shown.

### Example 11 (Comparative example)

### Manufacture of DES comprising choline chloride:urea (ratio 1:2 by mol)

86.0 g (1.43 mol) of urea and 100.0 g of choline chloride (0.72 mol) were put into plastic vessel and mixed thoroughly with a mechanical laboratory mixer at 80°C. A clear DES solution was formed in 1 hour.

### Example 12 (Comparative example)

### Dissolution of dissolving cellulose pulp in DES of example 11

2.79 g of anhydroys betaine (0.02 mol) was dissolved in 3.2 g (0.18 mol) of distilled water. 12.0 g of dried dissolving pulp (dry-matter content∼94 %) was immersed in 12.0 g of distilled water to activate the dry pulp. The betaine solution and activated pulp were added into the solution described in example 11. The open vessel was kept in a water-bath at 80°C and the slurry was vigorously stirred. Water was evaporated from the slurry until the water content was approximately 5 wt-% of the total DES solution weight (net weight of water, choline chloride and boric acid). This took approximately 2 hours. The cellulose concentration was approximately 5.3 wt-% of the total mass weight. After this the vessel was sealed and the stirring was continued during 2 hour. The final sample still contained separate phases of fibres and liquid and extensive cellulose dissolution was not observed. In Figure 2 a photo of the final sample is shown.

### Example 13

### Manufacture of DES comprising choline chloride:boric acid (ratio 3:2 by mol)

59.1 g (0.96 mol) of boric acid and 200.0 g of choline chloride (1.43 mol) were put into teflon vessel placed in a water-bath and mixed thoroughly with a mechanical laboratory mixer at 80°C. A clear DES solution was formed in 1 hour.

### Example 14

### Dissolution of dissolving cellulose pulp in DES of example 13

2.6 g of anhydroys betaine (0.019 mol) was dissolved in 3.4 g (0.19 mol) of distilled water. 30.0 g of dried dissolving pulp (dry-matter content∼94 %) was immersed in 30.0 g of distilled water to activate the dry pulp. The betaine solution and activated pulp were added into the solution described in example 13. The open vessel was kept in a water-bath at 80°C and the slurry was vigorously stirred. Water was evaporated from the slurry until the water content was approximately 5 wt-% of the total DES solution weight (net weight of water, choline chloride and boric acid). This took approximately 2 hours. The cellulose concentration was approximately 9.3 wt-% of the total mass weight. After this the vessel was sealed and the stirring was continued during 3 hour. A turbid very viscous dope containing some fibre particles was formed.

### Example 15

### Dissolution of birch pulp cellulose in DES comprising acetylcholine chloride: boric acid (ratio 3:2 by mol)

5.67 g (0.091 mol) of boric acid and 25.0 g (0.137 mol) of acetylcholine chloride were mixed thoroughly with a mechanical laboratory mixer. 1 ml of water (3% w/w) was added to the mixture and the temperature raised to 50°C. A clear solution is formed in 1-2 hours. 1.0g of birch pulp cellulose (0.031 mol of OH groups, 3% w/w) is added to the solution and temperature raised to 80 °C. A viscous solution is formed during 3-4 hours.

### Example 16

### Method for the manufacture of cellulose beads

DES consisting of 2:3 boric acid: choline chloride was prepared by combining 22.2 g of boric acid and 100g of choline chloride and heating the mixture at 80°C for 4-6 hours. 5 weight% of dissolving pulppulp was then solubilized to the DES by warming the mixture at 80°C for 8 hours. Then 5 weight% of water was added to this mixture and stirring continued at 80°C for one hour. The mixture was then forced using increased pressure dropwise through a 50 mm long needle with inner diameter of 0.8 mm into 2 M nitric acid at room temperature to obtain cellulose beads. The beads were left in nitric acid for 2 h to ensure complete coagulation and washed at least 4 h under running tap water and with several washing cycles using distilled water until the pH of the mixture was neutral. After the washing procedure, the beads remained inert to mixing and were not dissociated upon mechanical stirring.

### Example 17

### Method for the manufacture of cellulose absorbent

DES consisting of 2:3 (or 1:3) boric acid: choline chloride was prepared by combining 22.2 g of boric acid and 100g of choline chloride and heating the mixture at 80°C for 4-6 hours. 5 weight% of dissolving pulp was then solubilized to the DES by warming the mixture at 80°C for 8 hours. Then excess of water weight% of water was added to this mixture and stirred, whereby the cellulose material was precipitated, rinsed with water and then freeze-dried, and a water-insoluble "sponge" is obtained.

Product "sponge" samples were cut into specimens (∼5 mm × 5 mm × 5 mm) and dipped into 20 mL of dodecane for 5 s. Dodecane and silica oil are generally used as suitable model compounds for oil absorbency. The samples were then shaken manually for 5s, and the oil absorption capacity was then calculated from the mass of the oil adsorbed into the sample. The highest values were 43.2g (dodecane) and 39.4g (silica oil) using the eutectic mixture 2:3. When the material was squeezed, oil slowly drips away from the sample. The sample retains its form (2-3h), which could be enhanced with drying.

The material was also used to "fish" dodecane from water. It could be clearly seen that dodecane was attached into the material and water has no strong adsorption. Water is initially slightly adsorbed into the material, but when the material is squeezed lightly, water drips away but dodecane is mostly still attached in to the sample.

The present invention has been described herein with reference to specific embodiments. It is, however clear to those skilled in the art that the methods and products may be varied within the scope of the claims.

## Claims

1. A deep eutectic solvent, **characterized in that** the deep eutectic comprises a (2-R-ethyl)-trimethylammonium salt having chemical formula I 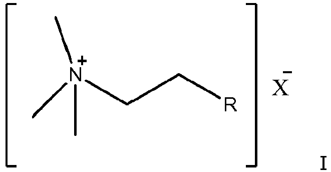 or a mixture of said salts where the group R is selected from OH, halogens, ester groups, ether groups and carbamoyl groups, and a Lewis acid selected from boric acid, meta-boric acid, boronic acid, borinic acid, alkyl borates and hydrated borate salts, and any combinations thereof, and the deep eutectic solvent comprises molar ratio from 5:1 to 1:1 of the (2-R-ethyl)-trimethylammonium salt and the Lewis acid, respectively.

2. The deep eutectic solvent according to claim 1, **characterized in that** the salt is an inorganic or organic salt, preferably a halogenide, acetate, lactate, butyrate or formiate.

3. The deep eutectic solvent according to claim 1 or 2, **characterized in that** the (2-R-ethyl)-trimethylammonium salt is selected from choline chloride, acetylcholine chloride, and chlorocholine chloride.

4. The deep eutectic solvent according to any one of claims 1-3, **characterized in that** the deep eutectic solvent comprises molar ratio from 3:1 to 1:1 of the (2-R-ethyl)-trimethylammonium salt and the Lewis acid, respectively.

5. The deep eutectic solvent according to any one of claims 1-4, **characterized in that** the deep eutectic solvent comprises 0-50 wt%, preferably 0.01-50 wt%, more preferably 2-15 wt% of water, calculated from the total weight of the deep eutectic solvent.

6. The deep eutectic solvent according to any one of claims 1-5, **characterized in that** the deep eutectic solvent comprises 0-50 wt%, preferably 0.01-50 wt%, more preferably 0.1-10 wt% and particularly preferably 0.1-3 wt% of a betaine compound, calculated from the total weight of the deep eutectic solvent.

7. The deep eutectic solvent according to claim 6, **characterized in that** the betaine compound is selected from betaine and betaine aldehyde.

8. A method for the manufacture of a deep eutectic solvent, **characterized in that** the method comprises the steps, where (2-R-ethyl)-trimethylammonium salt having chemical formula I 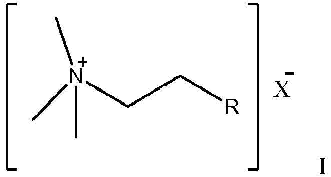 or a mixture of said salts where the group R is selected from OH, halogens,, ester groups and ether groups and carbamoyl groups, is mixed with a Lewis acid selected from boric acid, meta-boric acid, boronic acid, borinic acid, alkyl borates and hydrated borate salts, and any combinations thereof, in molar ratio from 5:1 to 1:1 of the (2-R-ethyl)-trimethylammonium salt and the Lewis acid, respectively.

9. The method according to claim 8, **characterized in that** the (2-R-ethyl)-trimethylammonium salt or a mixture of said salts is mixed with a Lewis acid selected from boric acid, meta-boric acid, boronic acid, borinic acid, alkyl borates and hydrated borate salts, and any combinations thereof, in molar ratio from 3:1 to 1:1 of the (2-R-ethyl)-trimethylammonium salt and the Lewis acid, respectively.

10. The method according to claim 8 or 9, **characterized in that** the salt is an inorganic or organic salt, preferably a halogenide, acetate, lactate, butyrate or formiate.

11. The method according to any one claims 8-10, **characterized in that** water is added before or after mixing (2-R-ethyl)-trimethylammonium salt or a mixture of said salts with the Lewis acid to obtain the water content of 0.01-50 wt%, preferably 2-15 wt% of water, calculated from the total weight of the deep eutectic solvent.

12. The method according to claim 11, **characterized in that** water is added in step-wise manner, preferably 50 wt% of water is added in the first step followed by mixing and adding the remaining 50 wt% in at least one step, followed by mixing.

13. The method according to any one claims 8-12, **characterized in that** 0.01-50 wt%, preferably 0.1-10 wt% and particularly preferably 0.1-3 wt% of a betaine compound, calculated from the total weight of the deep eutectic solvent, is added before or after mixing (2-R-ethyl)-trimethylammonium salt or a mixture of said salts with the Lewis acid.

14. The method according to claim 13, **characterized in that** the betaine compound is selected from betaine and betaine aldehyde.

15. The method according to any one claims 8-14, **characterized in that** the mixing is carried out at a temperature of 20-100°C, preferably 40-95°C, particularly preferably 50-90°C.

16. A method for dissolving polysaccharides and/or natural polyphenols, **characterized in that** the method comprises the steps, where 0.1 - 50 wt% of polysaccharide and/or natural polyphenol is mixed with 99.9 - 50 wt% of a deep eutectic solvent according to any one of claims 1-7, at a temperature of 20-150°C to obtain a polysaccharide solution.

17. The method according to claim 16, **characterized in that** 0.5-30 wt% of the polysaccharide and/or natural polyphenols is used.

18. The method according to claim 16 or 17, **characterized in that** the mixing temperature is 50-90°C.

19. The method according to any one of claims 16-18, **characterized in that** the polysaccharide is selected from cellulose, cellulose derivatives, starch, starch derivatives, xylanes, hemicelluloses, chitosans, pectins, maltodextrines, dextranes and lignocellulosic materials and combinations thereof.

20. The method according to any one of claims 16-19, **characterized in that** the natural polyphenol is selected from lignins and tannins and combinations thereof.

21. The method according to any one of claims 16-20, **characterized in that** a deep eutectic solvent according to any one of claims 1-5 is used and a betaine compounds selected from betaine and betaine aldehyde is added to the polysaccharide solution.

22. The method according to any one of claims 16-21, **characterized in that** the pH of the polysaccharide solution or the pH of the deep eutectic solvent according to any one of claims 1-7 is adjusted to the at least 7 with an organic or inorganic base, preferably the base is selected from alkali metal hydroxides, alkali metal borates, earth alkaline metal hydroxides, earth alkaline metal borates, ammonium hydroxide, ammonium borate and ammonia gas.

23. A method for the manufacture of cellulose beads, **characterized in that** the method comprises the steps where 0.1 - 50 wt% of cellulosic material is mixed with the deep eutectic solvent according to any one of claims 1-7, to obtain a cellulosic suspension, followed by adding 1-10 wt% of water and mixing to obtain an aqueous mixture, followed by forcing the aqueous mixture through a nozzle drop-wise to an aqueous nitric acid solution whereby cellulose beads are formed, and washing the cellulose beads with water.

24. A method for the manufacture of cellulose absorbent, **characterized in that** the method comprises the steps where 1-50 wt% of cellulosic material is mixed with the deep eutectic solvent according to any one of claims 1-7, to obtain a homogeneous cellulosic suspension, followed by adding water to the suspension, whereby a mixture comprising precipitation is formed, followed by filtrating the mixture and washing with water and freeze-drying the obtained solid material.

25. The method for the manufacture of cellulose absorbent according to claim 24, **characterized in that** methyltrimethoxysilane in a molar ratio of 0-10 with regard to anhydroglucose units is added to the cellulosic suspension prior to water addition.

## Patentansprüche

1. Stark eutektisches Lösungsmittel, **dadurch gekennzeichnet, dass** das stark eutektische Lösungsmittel ein (2-R-Ethyl)-Trimethylammoniumsalz mit der chemischen Formel I 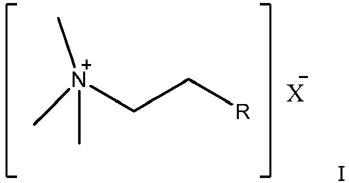 oder ein Gemisch dieser Salze, wobei die R-Gruppe aus OH, Halogenen, Estergruppen, Ethergruppen und Carbamoylgruppen ausgewählt ist, und eine Lewis-Säure ausgewählt aus Borsäure, Metaborsäure, Boronsäure, Borinsäure, Alkylboraten und hydratisierten Boratsalzen, und Kombinationen derselben, umfasst und das stark eutektische Lösungsmittel ein Molverhältnis des (2-R-Ethyl)-Trimethylammoniumsalzes und der Lewis-Säure von 5:1 bis 1:1 umfasst.

2. Stark eutektisches Lösungsmittel nach Anspruch 1, **dadurch gekennzeichnet, dass** das Salz ein anorganisches oder organisches Salz, vorzugsweise ein Halogenid, Acetat, Lactat, Butyrat oder Formiat, ist.

3. Stark eutektisches Lösungsmittel nach Anspruch 1 oder 2, **dadurch gekennzeichnet, dass** das (2-R-Ethyl)-Trimethylammoniumsalz aus Cholinchlorid, Acetylcholinchlorid und Chlorcholinchlorid ausgewählt ist.

4. Stark eutektisches Lösungsmittel nach einem der Ansprüche 1 bis 3, **dadurch gekennzeichnet, dass** das stark eutektische Lösungsmittel ein Molverhältnis des (2-R-Ethyl)-Trimethylammoniumsalzes und der Lewis-Säure von 3:1 bis 1:1 umfasst.

5. Stark eutektisches Lösungsmittel nach einem der Ansprüche 1 bis 4, **dadurch gekennzeichnet, dass** das stark eutektische Lösungsmittel 0-50 Gew.-%, bevorzugt 0,01-50 Gew.-%, bevorzugter 2-15 Gew.-% Wasser umfasst, berechnet in Bezug auf das Gesamtgewicht des stark eutektischen Lösungsmittels.

6. Stark eutektisches Lösungsmittel nach einem der Ansprüche 1 bis 5, **dadurch gekennzeichnet, dass** das stark eutektische Lösungsmittel 0-50 Gew.-%, bevorzugt 0,01-50 Gew.-%, bevorzugter 0,1-10 Gew.-% und besonders bevorzugt 0,1-3 Gew.-% einer Betainverbindung umfasst, berechnet in Bezug auf das Gesamtgewicht des stark eutektischen Lösungsmittels.

7. Stark eutektisches Lösungsmittel nach Anspruch 6, **dadurch gekennzeichnet, dass** die Betainverbindung aus Betain und Betainaldehyd ausgewählt ist.

8. Verfahren zur Herstellung eines stark eutektischen Lösungsmittels, **dadurch gekennzeichnet, dass** das Verfahren die Schritte umfasst, in denen ein (2-R-Ethyl)-Trimethylammoniumsalz mit der chemischen Formel I 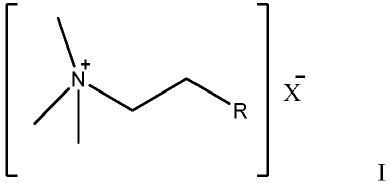 oder ein Gemisch dieser Salze, wobei die R-Gruppe aus OH, Halogenen, Estergruppen und Ethergruppen und Carbamoylgruppen ausgewählt ist, mit einer Lewis-Säure, die aus Borsäure, Metaborsäure, Boronsäure, Borinsäure, Alkylboraten und hydratisierten Boratsalzen, und Kombinationen derselben, ausgewählt ist, in einem Molverhältnis des (2-R-Ethyl)-Trimethylammoniumsalzes und der Lewis-Säure von 5:1 bis 1:1 gemischt wird.

9. Verfahren nach Anspruch 8, **dadurch gekennzeichnet, dass** das (2-R-Ethyl)-Trimethylammoniumsalz oder ein Gemisch dieser Salze mit einer Lewis-Säure, die aus Borsäure, Metaborsäure, Boronsäure, Borinsäure, Alkylboraten und hydratisierten Boratsalzen, und Kombinationen derselben, ausgewählt ist, in einem Molverhältnis des (2-R-Ethyl)-Trimethylammoniumsalzes und der Lewis-Säure von 3:1 bis 1:1 gemischt wird.

10. Verfahren nach Anspruch 8 oder 9, **dadurch gekennzeichnet, dass** das Salz ein anorganisches oder organisches Salz, vorzugsweise ein Halogenid, Acetat, Lactat, Butyrat oder Formiat, ist.

11. Verfahren nach einem der Ansprüche 8 bis 10, **dadurch gekennzeichnet, dass** vor oder nach dem Mischen des (2-R-Ethyl)-Trimethylammoniumsalzes, oder eines Gemischs dieser Salze, mit der Lewis-Säure Wasser zugegeben wird, um den Wassergehalt von 0,01-50 Gew.-%, bevorzugt 2-15 Gew.-% Wasser, zu erzeugen, berechnet in Bezug auf das Gesamtgewicht des stark eutektischen Lösungsmittels.

12. Verfahren nach Anspruch 11, **dadurch gekennzeichnet, dass** die Wasserzugabe stufenweise erfolgt, wobei bevorzugt 50 Gew.-% Wasser im ersten Schritt zugegeben werden, wonach gemischt wird, und die verbleibenden 50 Gew.-% in mindestens einem Schritt zugegeben werden, wonach gemischt wird.

13. Verfahren nach einem der Ansprüche 8 bis 12, **dadurch gekennzeichnet, dass** 0,01-50 Gew.-%, bevorzugt 0,1-10 Gew.-% und besonders bevorzugt 0,1-3 Gew.-% einer Betainverbindung, berechnet in Bezug auf das Gesamtgewicht des stark eutektischen Lösungsmittels, vor oder nach dem Mischen des (2-R-Ethyl)-Trimethylammoniumsalzes, oder eines Gemischs dieser Salze, mit der Lewis-Säure Wasser zugegeben werden.

14. Verfahren nach Anspruch 13, **dadurch gekennzeichnet, dass** die Betainverbindung aus Betain und Betainaldehyd ausgewählt ist.

15. Verfahren nach einem der Ansprüche 8 bis 14, **dadurch gekennzeichnet, dass** das Mischen bei einer Temperatur von 20-100 °C, bevorzugt 40-95 °C, besonders bevorzugt 50-90 °C, ausgeführt wird.

16. Verfahren zum Auflösen von Polysacchariden und/oder natürlichen Polyphenolen, **dadurch gekennzeichnet, dass** das Verfahren die Schritte umfasst, in denen 0,1-50 Gew.-% Polysaccharid und/oder natürliches Polyphenol mit 99,9-50 Gew.-% eines stark eutektischen Lösungsmittels aus einem der Ansprüche 1 bis 7 bei einer Temperatur von 20-150 °C gemischt werden, um eine Polysaccharid-Lösung zu erhalten.

17. Verfahren nach Anspruch 16, **dadurch gekennzeichnet, dass** 0,5-30 Gew.-% des Polysaccharids und/oder natürlichen Polyphenols verwendet werden.

18. Verfahren nach Anspruch 16 oder 17, **dadurch gekennzeichnet, dass** die Mischtemperatur 50-90 °C beträgt.

19. Verfahren nach einem der Ansprüche 16 bis 18, **dadurch gekennzeichnet, dass** das Polysaccharide ausgewählt ist aus Cellulose, Cellulosederivaten, Stärke, Stärkederivaten, Xylanen, Hemicellulosen, Chitosanen, Pektinen, Maltodextrinen, Dextranen und Lignocellulose-Materialien und Kombinationen derselben.

20. Verfahren nach einem der Ansprüche 16 bis 19, **dadurch gekennzeichnet, dass** das natürliche Polyphenol aus Ligninen und Tanninen und Kombinationen derselben ausgewählt ist.

21. Verfahren nach einem der Ansprüche 16 bis 20, **dadurch gekennzeichnet, dass** ein stark eutektisches Lösungsmittel aus einem der Ansprüche 1 bis 5 verwendet wird und der Polysaccharid-Lösung eine aus Betain und Betainaldehyd ausgewählte Betainverbindung zugegeben wird.

22. Verfahren nach einem der Ansprüche 16 bis 21, **dadurch gekennzeichnet, dass** der pH-Wert der Polysaccharid-Lösung oder der pH-Wert des stark eutektischen Lösungsmittels aus einem der Ansprüche 1 bis 7 mit einer organischen oder anorganischen Base auf mindestens 7 eingestellt wird, wobei die Base vorzugsweise aus Alkalimetallhydroxiden, Alkalimetallboraten, Erdalkalimetallhydroxiden, Erdalkalimetallboraten, Ammoniumhydroxid, Ammoniumborat und Ammoniakgas ausgewählt ist.

23. Verfahren zur Herstellung von Cellulose-Kügelchen, **dadurch gekennzeichnet, dass** das Verfahren die Schritte umfasst, in denen 0,1-50 Gew.-% eines Cellulosematerials mit dem stark eutektischen Lösungsmittels aus einem der Ansprüche 1 bis 7 gemischt werden, um eine Cellulose-Suspension zu erhalten, danach werden 1-10 Gew.-% Wasser zugegeben und gemischt, um ein wässriges Gemisch herzustellen, danach wird das wässrige Gemisch tröpfchenweise durch eine Düse zu einer wässrigen Salpetersäurelösung durchgepresst, wodurch Cellulose-Kügelchen gebildet werden, und die Cellulose-Kügelchen werden mit Wasser gewaschen.

24. Verfahren zur Herstellung eines Cellulose-Absorptionsmittels, **dadurch gekennzeichnet, dass** das Verfahren die Schritte umfasst, in denen 1-50 Gew.-% eines Cellulosematerials mit dem stark eutektischen Lösungsmittel aus einem der Ansprüche 1 bis 7 gemischt werden, um eine homogene Cellulose-Suspension zu erzeugen, wonach der Suspension Wasser zugegeben wird, wodurch ein fällungshaltiges Gemisch gebildet wird, gefolgt vom Filtrieren des Gemischs und Waschen mit Wasser und Gefriertrocknen des angefallenen Festmaterials.

25. Verfahren zur Herstellung eines Cellulose-Absorptionsmittels nach Anspruch 24, **dadurch gekennzeichnet, dass** der Cellulose-Suspension vor der Wasserzugabe Methyltrimethoxysilan in einem auf Anhydroglucose-Einheiten bezogenen Molverhältnis von 0-10 zugegeben wird.

## Revendications

1. Solvant eutectique profond, **caractérisé en ce que** le solvant eutectique profond comprend un sel de (2-R-éthyl)-triméthylammonium ayant la formule chimique I 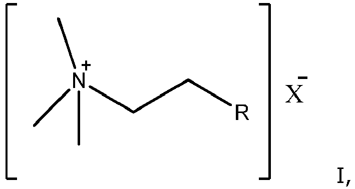 ou un mélange desdits sels, ledit groupe R étant choisi parmi OH, halogènes, groupes ester, groupes éther et groupes carbamoyl, et un acide de Lewis choisi parmi l'acide borique, l'acide métaborique, l'acide boronique, l'acide borinique, les borates d'alkyle et les sels de borate hydratés et leurs combinaisons, et que le solvant eutectique profond comprend un rapport molaire de 5:1 à 1:1 entre le sel de (2-R-éthyl)-triméthylammonium et l'acide de Lewis, respectivement.

2. Solvant eutectique profond selon la revendication 1, **caractérisé en ce que** le sel est un sel inorganique ou organique, préférablement un halogénure, acétate, lactate, butyrate ou formiate.

3. Solvant eutectique profond selon la revendication 1 ou 2, **caractérisé en ce que** le sel de (2-R-éthyl)-triméthylammonium est choisi parmi le chlorure de choline, le chlorure d'acétylcholine et le chlorure de chlorocholine.

4. Solvant eutectique profond selon l'une des revendications 1 à 3, **caractérisé en ce que** le solvant eutectique profond comprend un rapport molaire de 3:1 à 1:1 entre le sel de (2-R-éthyl)-triméthylammonium et l'acide de Lewis, respectivement.

5. Solvant eutectique profond selon l'une des revendications 1 à 4, **caractérisé en ce que** le solvant eutectique profond comprend de 0 à 50 % en poids, préférablement de 0,01 à 50 % en poids, plus préférablement de 2 à 15 % en poids d'eau, calculé par rapport au poids total du solvant eutectique profond.

6. Solvant eutectique profond selon l'une des revendications 1 à 5, **caractérisé en ce que** le solvant eutectique profond comprend de 0 à 50 % en poids, préférablement de 0,01 à 50 % en poids, plus préférablement de 0,1 à 10 % en poids, et particulièrement préférablement de 0,1 à 3 % en poids d'un composé de bétaïne, calculé par rapport au poids total du solvant eutectique profond.

7. Solvant eutectique profond selon la revendication 6, **caractérisé en ce que** le composé de bétaïne est choisi parmi la bétaïne et l'aldéhyde de bétaïne.

8. Procédé de fabrication d'un solvant eutectique profond, **caractérisé en ce que** le procédé comprend les étapes consistant à mélanger le sel de (2-R-éthyl)-triméthylammonium ayant la formule chimique I 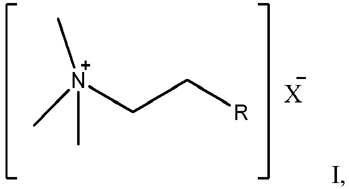 ou un mélange desdits sels, ledit groupe R étant choisi parmi les OH, halogènes, groupes ester et groupes éther et groupes carbamoyl, avec un acide de Lewis choisi parmi l'acide borique, l'acide métaborique, l'acide boronique, l'acide borinique, les borates d'alkyle et les sels de borate hydratés, et leurs combinaisons, dans un rapport molaire de 5:1 à 1:1 entre le sel de (2-R-éthyl)-triméthylammonium et l'acide de Lewis, respectivement.

9. Procédé selon la revendication 8, **caractérisé en ce que** le sel de (2-R-éthy)-triméthylammonium, ou un mélange desdits sels, est mélangé avec un acide de Lewis choisi parmi l'acide borique, l'acide métaborique, l'acide boronique, l'acide borinique, les borates d'alkyle et les sels de borate hydratés et leurs combinaisons dans un rapport molaire de 3:1 à 1:1 entre le sel de (2-R-éthyl)-triméthylammonium et l'acide de Lewis, respectivement.

10. Procédé selon la revendication 8 ou 9, **caractérisé en ce que** le sel est un sel inorganique ou organique, préférablement un halogénure, acétate, lactate, butyrate ou formiate.

11. Procédé selon l'une des revendications 8 à 10, **caractérisé en ce qu'**on ajoute de l'eau avant ou après le mélange du sel de (2-R-éthyl)-triméthylammonium, ou d'un mélange desdits sels, avec l'acide de Lewis pour obtenir la teneur en eau de 0,01 à 50 % en poids, préférablement de 2 à 15 % en poids d'eau, calculée par rapport au poids total du solvant eutectique profond.

12. Procédé selon la revendication 11, **caractérisé en ce qu'**on ajoute l'eau par étapes, 50 % en poids d'eau étant préférablement ajoutés dans la première étape, suivie d'un brassage, et les 50 % en poids restants étant ajoutés dans au moins une étape, suivie d'un brassage.

13. Procédé selon l'une des revendications 8 à 12, **caractérisé en ce qu'**on ajoute de 0,01 à 50 % en poids, préférablement de 0,1 à 10 % en poids, et particulièrement préférablement de 0,1 à 3 % en poids d'un composé de bétaïne, calculé par rapport au poids total du solvant eutectique profond, avant ou après le mélange du sel de (2-R-éthyl)-triméthylammonium, ou d'un mélange desdits sels, avec l'acide de Lewis.

14. Procédé selon la revendication 13, **caractérisé en ce que** le composé de bétaïne est choisi parmi la bétaïne et l'aldéhyde de bétaïne.

15. Procédé selon l'une des revendications 8 à 14, **caractérisé en ce que** le mélange est effectué par une température de 20 à 100 °C, préférablement de 40 à 95 °C, particulièrement préférablement de 50 à 90 °C.

16. Procédé de dissolution de polysaccharides et/ou de polyphénols naturels, **caractérisé en ce que** le procédé comprend les étapes consistant à mélanger de 0,1 à 50 % en poids de polysaccharide et/ou de polyphénol naturel avec 99,9 à 50 % en poids d'un solvant eutectique profond selon l'une des revendications 1 à 7 par une température de 20 à 150 °C pour obtenir une solution de polysaccharides.

17. Procédé selon la revendication 16, **caractérisé en ce qu'**on utilise de 0,5 à 30 % en poids du polysaccharide et/ou des phénols naturels.

18. Procédé selon la revendication 16 ou 17, **caractérisé en ce que** la température de mélange est de 50 à 90 °C.

19. Procédé selon l'une des revendications 16 à 18, **caractérisé en ce que** les polysaccharides sont choisis parmi la cellulose, les dérivés de cellulose, l'amidon, les dérivés d'amidon, les xylanes, les hémicelluloses, les chitosanes, les pectines, les maltodextrines, les dextranes et les matières lignocellulosiques et leurs combinaisons.

20. Procédé selon l'une des revendications 16 à 19, **caractérisé en ce que** le polyphénol naturel est choisi parmi les lignines et les tanins et leurs combinaisons.

21. Procédé selon l'une des revendications 16 à 20, **caractérisé en ce qu'**un solvant eutectique profond selon l'une des revendications 1 à 5 est utilisé et un composé de bétaïne choisi parmi la bétaïne et l'aldéhyde de bétaïne est ajouté à la solution de polysaccharides.

22. Procédé selon l'une des revendications 16 à 21, **caractérisé en ce que** la valeur pH de la solution de polysaccharides ou la valeur pH du solvant eutectique profond selon l'une des revendications 1 à 7 est ajustée par une base organique ou inorganique à au moins 7, ladite base étant préférablement choisie parmi les hydroxydes de métaux alcalins, les borates de métaux alcalins, les hydroxydes de métaux alcalinoterreux, les borates de métaux alcalinoterreux, l'hydroxyde d'ammonium, le borate d'ammonium et le gaz ammoniacal.

23. Procédé de fabrication de billes de cellulose, **caractérisé en ce que** le procédé comprend les étapes consistant à mélanger de 0,1 à 50 % en poids d'une matière cellulosique avec le solvant eutectique profond selon l'une des revendications 1 à 7 pour obtenir une solution cellulosique, suivie par l'étape consistant à ajouter de 1 à 10 % en poids d'eau et à brasser pour obtenir un mélange aqueux, puis à forcer le mélange aqueux, goutte à goutte, à travers une buse d'eau vers une solution aqueuse d'acide nitrique, ce qui cause la formation de billes de cellulose, et à laver les billes de cellulose avec de l'eau.

24. Procédé de fabrication d'un absorbant cellulosique, **caractérisé en ce que** le procédé comprend les étapes consistant à mélanger de 1 à 50 % en poids d'une matière cellulosique avec le solvant eutectique profond selon l'une des revendications 1 à 7 pour obtenir une solution cellulosique homogène, puis à ajouter de l'eau à la suspension, ce qui cause la formation d'un mélange comprenant un précipité, puis à filtrer le mélange et à le laver à l'eau et à lyophiliser la matière solide obtenue.

25. Procédé de fabrication d'un absorbant cellulosique selon la revendication 24, **caractérisé en ce qu'**on ajoute du méthyltriméthoxysilane à la suspension cellulosique dans un rapport molaire de 0-10 par rapport aux unités anhydroglucose avant d'ajouter l'eau.
